(19) Europäisches Patentamt / European Patent Office / Office européen des brevets

(11) **EP 2 125 213 B1**

(12) **EUROPÄISCHE PATENTSCHRIFT**

(45) Veröffentlichungstag und Bekanntmachung des Hinweises auf die Patenterteilung:
**20.03.2019 Patentblatt 2019/12**

(21) Anmeldenummer: **08708303.6**

(22) Anmeldetag: **28.01.2008**

(51) Int Cl.:
*B01J 35/08* (2006.01)     *B01J 37/08* (2006.01)
*C07C 45/35* (2006.01)     *C07C 51/25* (2006.01)
*B01J 27/199* (2006.01)     *B01J 23/887* (2006.01)
*B01J 23/888* (2006.01)     *B01J 23/889* (2006.01)
*B01J 23/89* (2006.01)

(86) Internationale Anmeldenummer:
**PCT/EP2008/050985**

(87) Internationale Veröffentlichungsnummer:
**WO 2008/104432 (04.09.2008 Gazette 2008/36)**

(54) **AUS HOHLEN FORMEN BESTEHENDE MISCHOXIDKATALYSATOREN**

MIXED OXIDE CATALYSTS MADE OF HOLLOW SHAPES

CATALYSEURS OXYDES MIXTES CONSTITUÉS DE FORMES CREUSES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **01.03.2007 DE 102007009981**

(43) Veröffentlichungstag der Anmeldung:
**02.12.2009 Patentblatt 2009/49**

(73) Patentinhaber: **Evonik Degussa GmbH**
**45128 Essen (DE)**

(72) Erfinder:
• **FISCHER, Achim**
  **63739 Aschaffenburg (DE)**
• **BURKHARDT, Werner**
  **63636 Brachttal (DE)**
• **RÖDER, Stefan**
  **36391 Sinntal (DE)**
• **HUTHMACHER, Klaus**
  **63571 Gelnhausen (DE)**

(74) Vertreter: **Evonik Patent Association**
**c/o Evonik Industries AG**
**IP Management**
**Bau 1042A/PB 15**
**Paul-Baumann-Straße 1**
**45764 Marl (DE)**

(56) Entgegenhaltungen:
**DE-A1- 10 344 149     GB-A- 1 442 566**
**GB-A- 2 055 787     JP-A- 5 277 381**
**US-A- 4 537 874**

EP 2 125 213 B1

**Beschreibung**

Die Erfindung betrifft aus hohlen Formen bestehende Mischoxidkatalysatoren für die katalytische Gasphasenoxidation von Olefinen, Verfahren zur Herstellung der Katalysatoren und die Umsetzung zu Aldehyden und Carbonsäuren mit Luft oder Sauerstoff in Gegenwart von inerten Gasen in unterschiedlichen Mengenverhältnissen, bei erhöhten Temperaturen und Drücken.

Insbesondere kann der Katalysator zur Umsetzung der stark exothermen Reaktion von Propen zu Acrolein und Acrylsäure oder Isobuten zu Methacrolein und Methacrylsäure eingesetzt werden. Die stark exotherme Umsetzung des Olefins an heterogenen Katalysatoren mit einem Sauerstoff enthaltenden Gas führt neben dem erwünschten Produkt Acrolein und Acrylsäure zu einer Reihe von Nebenprodukten: beispielsweise zur Bildung von $CO_2$, CO, Acetaldehyd oder Essigsäure.

Es ist bekannt, dass durch Art der chemischen Zusammensetzung des Mischoxides (Phasenbildung und Bildung von Reaktionszentren) wie auch durch Art des physikalischen Aufbaus (z. Bsp. Porosität, Oberflächengröße, Form des Katalysators), die Art der Wärmeabfuhr, die Fähigkeit zur Produktbildung (Selektivität) und der Produktivität (Raum - Zeitausbeute) stark beeinflusst werden kann. Im Fall der Olefin-Oxidation werden als Katalysator in der Regel Mischoxide eingesetzt, die in ihrem chemischen und physikalischen Aufbau eine komplexe Struktur aufweisen. Eine Vielzahl von Publikationen beschreibt Mischoxide, die in der Lage sind, als Katalysatoren für die Herstellung von Acrolein und Acrylsäure aus Propen eingesetzt zu werden. Diese Katalysatoren bestehen in der Regel aus Molybdän, Vanadium und/oder Wolfram. Zu diesen Basiskomponenten werden in der Regel mindestens eines der Elemente Wismut, Antimon, Vanadium, Tellur, Zinn, Eisen, Cobalt, Nickel und/oder Kupfer zugegeben.

Die Anzahl der Publikationen zur heterogen katalysierten Gasphasenoxidation von Olefinen zu Acrolein und Acrylsäure ist seit der ersten Entwicklung GB 821999 (1958) bei der Standard Oil Inc. zahlreich. Trotz der langen Entwicklungszeit, ist es noch immer eine anspruchsvolle Aufgabe, die Leistung des Katalysators wie Produktausbeute, Aktivität und Lebensdauer zu verbessern. Hierzu werden in der Literatur diverse Techniken zur Herstellung, sowie Rezepturen des Katalysators beansprucht. Exemplarisch seien hier die neusten Entwicklungen dargestellt:

STAND DER TECHNIK

Die WO 2005/063673 betrifft die Verdünnung des Katalysators durch ein inertes Material, um die Wärmebildung in der Reaktionszone zu reduzieren und dadurch die Produktausbeute zu erhöhen. Durch Vermeiden einer zu hohen Übertemperatur wird die Totaloxidation der Produkte reduziert. Trotz der Temperaturmodulation der Reaktion durch Inerte, erreicht das man mit dem beschriebenen Verfahren nur eine Summenausbeute an Acrolein und Acrylsäure von maximal 91,22 %.

In der WO2005035115 wird die Herstellung eines Katalysators der Zusammensetzung Metalloxid und sublimierbaren Materialien beschrieben. Das Metalloxid wirkt katalytisch, das sublimierbare Material als Additiv zur Porengenerierung. Der daraus resultierende Katalysator ist sehr aktiv, besitzt eine große Oberfläche und ist in der Lage Acrolein und Acrylsäure mit hoher Selektivität zu bilden.

Aus der DE 10344149 ist ein ringförmiger Vollkatalysator auf der Basis eines $Mo_{12}Bi_aFe_bX1_cX2_dX3_eX4_fO_n$ (1) mit einer Länge von 2-11 mm, einem äußeren Durchmesser von 2-11 mm und einer Wandstärke von 0.75-1.75 mm zur partiellen Oxidation von Propen zu Acrolein oder Methacrolein bekannt. Der Katalysator (I) hat den Vorteil einer verbesserten Aktivität und Selektivität.

In der DE 199 33450 werden Metallkatalysatoren aus Nickel, die hohle Formen oder Kugeln umfassen aus einer Metalllegierung beschrieben, die zur Hydrierung, Dehydrierung, Isomerisierung, reduktiven Alkylierung und reduktiven Aminierung eingesetzt werden. Der so hergestellte Katalysator besitzt eine verbesserte Festigkeit und Lebensdauer.

Derartige Hohlkugeln können gemäß Andersen, Schneider und Stephanie (vgl. "neue Hochporöse Metallische Werkstoffe", Ingenieur-Werkstoffe, 4, 1998, S. 36-38) hergestellt werden. Bei diesem Verfahren wird ein Gemisch der erwünschten Legierung, eines organischen Bindemittels und wahlweise eines anorganischen Bindemittels gleichmäßig durch ein Fließbett aus Polystyrolkugeln gesprüht, wo es die Kugeln beschichtet. Die beschichteten Kugeln werden sodann bei wahlweisen Temperaturen im Bereich von 450 bis 1000°C kalziniert, um das Polystyrol herauszubrennen, gefolgt von einer höheren Kalzinierungstemperatur, um das Metall zusammenzusintern und die Hohlform stabiler zu machen. Nach dem Kalzinieren wird der Katalysator durch Natronlauge aktiviert, um den aktivierten Grundmetallkatalysator herzustellen. Ein zusätzlicher Vorteil dieses Katalysatorsystems ist, dass man die Dicke der Wände der Hohlformen durch die Beschichtungsbedingungen und die Porosität der Wand durch die Teilchengröße und Zusammensetzung des ursprünglichen Pulvergemischs leicht steuern kann.

In der US 4,537,874 werden Mischoxidkatalysatoren auf Basis von Molybdän und Bismut offenbart, die zu Ringen geformt und als Katalysatoren in der Herstellung von ungesättigten Aldehyden eingesetzt werden. Die deutsche Offenlegungsschrift DE 103 44 149 A1 offenbart ebenfalls ringförmige Vollkatalysatoren, deren Aktivmasse ein Mischoxid auf Basis von Molybdän und Bismut ist. Mischoxidkatalysatoren in Form von Hohlkugeln werden in diesen Dokumenten jedoch nicht offenbart.

Die japanische Offenlegungsschrift JP 05 277381 beschreibt die Herstellung von auf Molybdän und Bismut basierenden Mischoxidkatalysatoren in Form von durch Sprühtrocknung erhältlichen hohler Kugeln von 5 bis 100 $\mu$m Durchmesser.

GB 2 055 787 A offenbart die Herstellung von keramischen Hohlkugeln umfassend das Auftragen auf einen organischen Träger und das anschließende Entfernen dieses organischen Trägers. Die Herstellung von aus hohlen Formen bestehenden Mischoxidkatalysatoren ist aus diesem Dokument jedoch nicht bekannt.

Ein Verfahren zur Herstellung eines aus stabilen Hohlkugeln bestehenden Materials als Träger für Katalysatoren zur Reinigung der Abgase von Verbrennungsmotoren ist aus der GB 1 442 566 A bekannt.

Aufgabe

Es ist allgemein akzeptiert, dass durch Art der chemischen Zusammensetzung des Mischoxides (Phasenbildung und Bildung von Reaktionszentren) wie auch durch Art des physikalischen Aufbaus (z. Bsp. Porosität, Oberflächengröße, Form des Katalysators), die Art der Wärmeabfuhr, die Fähigkeit zur Produktbildung (Selektivität) und der Produktivität (Raum - Zeitausbeute) stark beeinflusst werden kann. Die vorliegende Erfindung hat zur Aufgabe, einen Katalysator mit einer erhöhten katalytischen Aktivität gegenüber dem Stand der Technik bereitzustellen.

Der Erfindung liegt ferner die Aufgabe zu Grunde, ein verbessertes Verfahren zur Herstellung von Aldehyden und Säuren bereitzustellen, bei dem Acrolein und Acrylsäure aus Propen durch Oxidation mit Luft oder Sauerstoff in Gegenwart von inerten Gasen, darunter Wasserdampf oder Abgasen aus der Reaktion bei erhöhten Temperaturen und der Gegenwart eines heterogenen Mischoxidkatalysators hergestellt werden. Es soll ein Mischoxidkatalysator bereitgestellt werden, mit dem neben Propenumsätzen größer 95% auch eine hohe Produktselektivität von größergleich 88% erreicht wird, so dass sich die Wirtschaftlichkeit des Verfahrens verbessert.

Beschreibung der Erfindung

Gegenstand der Erfindung sind aus hohlen Formen bestehende Mischoxidkatalysatoren der allgemeinen Formel

$$(Mo_{12}Bi_aC_b(Co+Ni)_cD_dE_eF_fG_gH_h)O_x \qquad (I),$$

in der bedeuten:

C: Eisen,
D: mindestens eines der Elemente ausgewählt aus der Gruppe W, P,
E: mindestens eines der Elemente ausgewählt aus der Gruppe Li, K, Na, Rb, Cs, Mg, Ca, Ba, Sr,
F: mindestens eines der Elemente ausgewählt aus der Gruppe Ce, Mn, Cr, V,
G: mindestens eines der Elemente ausgewählt aus der Gruppe Nb, Se, Te, Sm, Gd, La, Y, Pd, Pt, Ru, Ag, Au,
H: mindestens eines der Elemente ausgewählt aus der Gruppe Si, Al, Ti, Zr,
und
a = 0,5 - 5,0
b = 0,5 - 5,0
c = 2 - 15
d = 0,01 - 5,0
e = 0,001 - 2
f = 0,001 - 5
g = 0 - 1,5
h = 0 - 800,
x = Zahl, die von der Wertigkeit und Häufigkeit der von Sauerstoff verschiedenen Elemente bestimmt wird, umfassend Hohlkugeln mit einem Durchmesser von 0,5 bis 5 mm.

In einer Ausführungsform umfasst der erfindungsgemäße Mischoxidkatalysator eine gasundurchlässige hohle Form.

In einer alternativen Ausführungsform umfasst der erfindungsgemäße Mischoxidkatalysator eine gasdurchlässige hohle Form.

In einer weiteren Ausführungsform umfasst der erfindungsgemäße Mischoxidkatalysator eine aus mehreren Schichten bestehende hohle Form.

Die Verwendung der erfindungsgemäßen Katalysatoren führt zu einer deutlich verbesserten Katalysatoraktivität, die sich darin äußert, dass geringere Salzbadtemperaturen für hohe Umsätze eingestellt werden können. Durch das neue Verfahren zur Herstellung der Katalysatoren der allgemeinen Formel I kann ein besonders geeigneter katalytisch aktiver Festkörper z.B. zur Umsetzung von Propen zu Acrolein und Acrylsäure erhalten werden. Besonders vorteilhaft führt man die Umsetzung in Reaktoren durch, die es erlauben, den Katalysator als Festbett einzusetzen. Es ist aber ebenfalls möglich, den Katalysator als Wirbelbettkatalysator einzusetzen. An dieser Stelle sei bemerkt, dass die erfindungsgemäßen Katalysatoren auch zur Umsetzung von iso-Buten zu Methacrolein und Methacrylsäure benutzt werden können.

Die Herstellung von Katalysatoren der beschriebenen Zusammensetzung kann dadurch erfolgen, dass man ein feinverteiltes Pulver durch die Fertigungsschritte: Lösen der Metallsalze, Fällung der aktiven Komponenten, Trocknung und Kalzination erzeugt und das kalzinierte Pulver in Form bringt. Dies kann wie allgemein bekannt durch Tablettierung, Extrusion oder durch Beschichtung eines Trägers geschehen. Die Trägerform ist nicht limitierend. So kann der Träger beispielsweise eine Pyramide, einen Zylinder oder eine Kugel sein.

Es wurde nun ein neues Verfahren gefunden, um den Mischoxidkatalysator eine hohle Form zu geben. Hierbei ist der Träger eine Matrix, die der Aktivmasse eine Form vorgibt, und nach oder während der Verfestigung der Aktivmasse entfernt wird, so dass ein Hohlkörper entsteht. Das Entfernen erfolgt durch gezieltes Herauslösen mittels eines Lösungsmittels oder bevorzugt thermisch, beispielsweise durch Wärmestrahlung. Dabei sollte der beschichtete Träger bevorzugt im Temperaturbereich von 450 bis 600°C in Gegenwart von Sauerstoff, insbesondere von Luft behandelt werden, so dass sich die katalytisch aktive Masse für den Einsatz in industriellen Reaktoren verfestigt und der Träger sich rückstandsfrei zersetzt. Als Träger werden organische Materialien, beispielsweise Polystyrol basierte Kunststoffe wie ASA (Acrylnitril/Styrol/Acrylester), Polystyrol (PS, PS-I), SAN (Styrol/Acrylnitril) eingesetzt. Es besteht jedoch keine Beschränkung auf diese Kunststoffe. Diese Materialien sind im allgemeinen deutlich billiger als die keramischen Träger, so dass sich die Herstellkosten des Katalysators reduzieren.

Ein weiterer Gegenstand der vorliegenden Erfindung ist daher ein Verfahren zur Herstellung von aus hohlen Formen bestehenden Mischoxid-Katalysatoren gemäß der vorliegenden Erfindung, umfassend, dass man Lösungen von Verbindungen der in den Mischoxidkatalysatoren gemäß Formel I enthaltenen Elemente vermischt, Kopräzipitate herstellt, den erhaltenen Feststoff isoliert, gegebenenfalls trocknet und kalziniert, und den erhaltenen feinteiligen Feststoff wahlweise zusammen mit einem Bindemittel in Form einer Suspension als Schicht auf einen Träger, der aus organischem Material besteht und die Form einer Kugel hat, aufbringt, und dieses organische Material nach oder während der Verfestigung der auf dieses aufgebrachte aufgebrachten Schicht entfernt.

Die Größe des Trägers ist nicht limitierend. Üblicherweise werden Körper von 0,1 bis 5 mm eingesetzt. Der so hergestellte Katalysator besitzt eine exzellente Aktivität bei hoher Selektivität und Lebensdauer und führt zu einer sehr guten Produktausbeute.

Die in dem beschriebenen Verfahren zur Gasphasenoxidation einzusetzenden Katalysatoren werden erhalten, indem die gelösten Verbindungen der katalytisch aktiven Elemente aus der Formel I mit den gewünschten Konzentrationen vereinigt werden. Die Komponenten werden idealerweise in Form der Verbindungen ausgewählt aus der Gruppe Ammonium- oder Aminverbindungen, Oxalate, Carbonate-, Phosphate-, Acetate, Carbonyle und/oder Nitrate einzeln oder gemeinsam eingesetzt. Besonders bevorzugt sind Carbonate, Nitrate und Phosphate oder Mischungen aus diesen. Ebenfalls eingesetzt werden können Säuren der Salze, beispielsweise Salpetersäure, Phosphorsäure oder Kohlensäure.

Die erste Stufe der Katalysatorherstellung bildet, wie bereits erwähnt, eine Fällung. Abhängig von der Art der Metallsalze, die in der Fällstufe eingesetzt werden, kann es erforderlich sein, die Komponenten in Form von Lösungsgemischen dem Fällungsgemisch zusetzen. Idealerweise verwendet man hierbei Ammoniak oder Ammonium-Salze, beispielsweise Ammoniumcarbonat, Ammoniumheptamolybdat oder Metallnitrate, beispielsweise Eisennitrat, Cobaltnitrat; man kann ebenfalls die entsprechenden Säuren, beispielsweise Salpetersäure in den zur Einstellung des Ionenverhältnisses notwendigen Mengen einsetzen. Der pH-Wert während der Fällung liegt bei < 8, insbesondere < 7.

Die Herstellung von Kopräzipitaten kann in einer Fällstufe durchgeführt werden. Besonders bevorzugt ist es, die Fällung mehrstufig durch schrittweise Zugabe der Einzelkomponenten oder durch Mischungen aus diesen durchzuführen. Die Anzahl der Fällstufen ist prinzipiell nicht limitiert. Bevorzugt sind aber ein bis drei Fällstufen.

Die erhaltene Suspension kann direkt weiterverarbeitet werden oder man lässt sie für > 0 bis 24 Stunden reifen, bevorzugt sind > 0 bis 12 Stunden, besonders bevorzugt sind > 0 bis 6 Stunden. Es ist selbstverständlich, dass die Fällsuspension vor der Weiterverarbeitung beispielsweise durch Rühren homogenisiert wird.

Nach der Reifung kann die Flüssigkeit aus der Suspension durch Verdampfung, Zentrifugation oder Filtration entfernt werden. Die Flüssigkeit zu verdampfen und gleichzeitig den Feststoff zu trocknen, ist ebenfalls möglich und kann beispielsweise durch Sprühtrocknung erfolgen. Die Flüssigkeit sollte bei einer Temperatur von 80 bis 130°C verdampft werden. Die Trocknung des Feststoffes kann mit Luft, sauerstoffhaltigen Inertgasen oder Inertgasen, beispielsweise Stickstoff erfolgen. Führt man die Trocknung in einem Ofen durch, so sollte die Temperatur zwischen 100 und 200°C liegen. In einem Sprühtrockner sollte die Anfangstemperatur der Trockenmediums von 200 bis 500°C und eine Temperatur bei Abscheidung des getrockneten Pulvers von 80 bis 200 °C vorsehen. Das erhaltene Korn sollte bevorzugt eine Korngrößenverteilung von 15 bis 160 $\mu$m mit einem mittleren Korndurchmesser zwischen 15 und 80 $\mu$m besitzen.

Das getrocknete Pulver kann prinzipiell anschließend in den unterschiedlichsten Ofentypen wie z. Bsp. in einem Umluftofen, Drehrohr, Hordenofen, Schachtofen oder Bandofen kalziniert werden. Die Regelgüte bzw. die Güte der Temperaturerfassung des Ofens sollte möglichst hoch sein. Die Verweildauer des Pulvers im Ofen sollte je nach Ofentyp zwischen 0,25 und 13 h betragen.

Ebenfalls ist es möglich, die Kalzinierung und die dabei eintretende thermische Zersetzung der Salze wie z. B. Nitraten oder Carbonaten in einer oder mehreren Stufen durchzuführen. Dabei können Temperaturen von 200 bis 600 °C angewandt, insbesondere 300° bis 600° benutzt werden. Die thermische Zersetzung kann unter Zusatz von Inertgas, aus Gemischen von Sauerstoff mit einem Inertgas durchgeführt werden.

Als Inertgas einsetzbar sind z. B. Stickstoff, Helium, Wasserdampf oder Gemische dieser Gase.

Das so erhaltene Pulver kann bereits als Katalysator eingesetzt werden. Die mittlere Korngrößenverteilung des Pulvers sollte 0,01 bis 50 $\mu$m reichen.

Um das Mischoxidpulver in die erfindungsgemäße Form zu überführen, wird es auf einen Träger aufgebracht, der nach der Verfestigung der katalytisch aktiven Masse entfernt wird, so dass ein Hohlkörper entsteht. Das Entfernen wird durch ein gezieltes Herauslösen mittels eines Lösungsmittels oder bevorzugt thermisch, beispielsweise durch Wärmestrahlung, durchgeführt. Dabei wird das aus Träger und katalytisch wirksamer Schicht bestehende Vorprodukt des erfindungsgemäßen Katalysators bevorzugt im Temperaturbereich von 490 bis 600°C, insbesondere 490 bis 580°C behandelt, so dass sich die katalytisch aktive Masse für den Einsatz in industriellen Reaktoren verfestigt und der Träger gleichzeitig oder anschließend rückstandsfrei entfernt. Als Träger werden organische Materialien, beispielsweise Polystyrol basierte Kunststoffe wie ASA (Acrylnitril/Styrol/Acrylester), Polystyrol (PS, PS-I), SAN (Styrol/Acrylnitril) eingesetzt, es besteht jedoch keine Beschränkung auf diese Kunststoffe, es können beispielsweise auch Cellulosen oder Zucker eingesetzt werden.

Nicht limitierend ist die Größe des Trägers. Üblicherweise werden Träger von 0,1 bis 5 mm eingesetzt. Die Dicke der Mischoxidbelegung liegt je nach Trägergröße in der Regel zwischen $10^{-6}$ und 1,5 mm, besonders bevorzugt ist eine Belegungsdicke von 0,1 bis 1,5 mm. Die Beschichtung des Trägers zur Herstellung des Katalysatorvorprodukts wird durch Versprühen einer wässrigen Suspension, die das Katalysatorpulver und Bindemittel enthält, durchgeführt. Das Katalysatorpulver wird bevorzugt in bei 470°C bis 600°C kalzinierter Form eingesetzt. Zur späteren Bildung von Poren kann der Suspension auch einer der bekannten Porenbildner zugesetzt werden.

Als Bindemittel können diverse Öle, Cellulosen, Polyvinylalkohole, Sacharide, Acrylate sowie Alkyl-Derivate, Mischungen oder Kondensate aus denselben eingesetzt werden. Bevorzugt sind Acrylate, Polyvinylalkohole sowie Cellulosen oder Zucker. Besonders bevorzugt sind Derivate und Kondensate von Acrylaten und/oder Cellulosen und/oder Zucker, sowie Mischungen aus diesen.

Nach der Trocknung des beschichteten Trägers bei Temperaturen von bevorzugt bis zu 110°C, wird der Träger entfernt. Das Entfernen wird durch ein gezieltes Herauslösen mittels eines geeigneten Lösungsmittels oder thermisch, beispielsweise durch Wärmestrahlung, bei erhöhten Temperaturen in Anwesenheit von Sauerstoff durchgeführt. Dabei sollte der beschichtete Träger bevorzugt im Temperaturbereich von 490 bis 600°C behandelt werden, so dass die Aktivmasse eine feste Schale bildet, während der Träger sich rückstandsfrei löst bzw. zersetzt.

Gegenstand der Erfindung ist ebenso ein Verfahren zur Herstellung von Aldehyden und Säuren durch Oxidation von Olefinen oder methylierten Aromaten mit Luft oder Sauerstoff in Gegenwart von Inertgasen, Wasserdampf oder Abgasen aus der Reaktion bei erhöhten Temperaturen in Gegenwart der erfindungsgemäßen Katalysatoren.

In einer Ausführungsform des erfindungsgemäßen Verfahrens zur Herstellung von Aldehyden und Säuren werden Katalysatoren gemäß den Ansprüchen 1 bis 4 eingesetzt.

In einer Ausführungsform des erfindungsgemäßen Verfahrens zur Herstellung von Aldehyden und Säuren gewinnt man Acrolein und Acrylsäure aus Propen.

In einer weiteren Ausführungsform des erfindungsgemäßen Verfahrens zur Herstellung von Aldehyden und Säuren gewinnt man Methacrolein und Methacrylsäure aus Isobuten.

In einer anderen Ausführungsform des erfindungsgemäßen Verfahrens zur Herstellung von Aldehyden und Säuren gewinnt man Benzaldehyd und Benzoesäure aus Toluol.

Die Reaktion zur Herstellung von Acrolein und Acrylsäure wird im allgemeinen bei Temperaturen von 250 - 450 °C und einem Druck von 1,0 - 2,2 bara durchgeführt. Dabei werden die Reaktionspartner Olefin, Luft und Inertgase bevorzugt im Verhältnis 1 : 6 - 9 : 3 - 18 bei einer Belastung von 2 - 10 mol Olefin/ dm$^3$ Katalysatorschüttung/h der Katalysatorschüttung zugeführt.

Anstelle von Inertgas kann das Abgas aus der Reaktion verwendet werden, aus dem die kondensierbaren Bestandteile abgetrennt wurden. Besonders gute Ergebnisse werden beim Einsatz von Rohrbündel- oder Wirbelbettreaktoren erzielt.

Die erfindungsgemäßen Katalysatoren führen auch bei hoher spezifischer Belastung zu einer verbesserten Aktivität bei der Verwendung in den genannten Oxidationsverfahren.

Die Erfindung wird nachfolgend anhand von Ausführungsbeispielen erläutert. Dabei ist definiert die Ausbeute (%) des Produktes als

$$\text{(mol/h Produkt gebildet) / (mol/h Reaktant zugeführt)} * 100$$

der Umsatz des Olefins (%) als

$$[1 - \text{(mol/h aus dem Reaktionsrohr austretendes Olefin)}/\text{(mol/h in das Reaktionsrohr eintretendes Olefin)}] * 100$$

die Selektivität (%) als

$$\text{(Ausbeute des Produktes / Umsatz)} * 100$$

Die dargestellte Erfindung wird um das Verständnis zu verbessern durch die nachstehenden Beispiele beschrieben, sie ist jedoch nicht auf diese Beispiele beschränkt.

BEISPIELE

Beispiel 1

Eine Lösung I wurde hergestellt, indem die Nitrate von Eisen, Cobalt, Nickel, Mangan, Kalium in den Massenanteilen 23,2 : 47,26 : 29,28 : 0,0646 : 0,2067 in 3,5 Liter Wasser gelöst, unter Rühren auf 40°C erhitzt und eine salpetersaure Lösung aus 0,1 mol $Sm^{3+}$ und 2 mol $HNO_3$ zugegeben wurde.

Für eine Lösung II wurde bei 40°C eine Lösung von 2118,6 g Ammoniumheptamolybdat in 2,7 l Wasser bereitet, dazu wurde 4,4 g Phosphorsäure sowie 0,42g Aerosil 200 (Degussa), 14g Aluminiumoxid in 1 l Wasser gegeben.

Lösung II wurde langsam und unter intensivem Rühren zu Lösung I gegeben. In einem getrennten Gefäß wurde ein weitere Lösung III bestehend aus 790 g Bismutnitrat und 0,72 mol $HNO_3$ angesetzt. Durch Zugabe dieser Lösung zu den anderen Aktivkomponenten wurde das Kopräzipität für die Herstellung der aktiven Katalysatorphase erhalten.

Das Kopräzipitat wurde 12 Stunden intensiv gerührt. Die erhaltene Suspension wurde in einem Sprühtrockner mit Drehscheibe bei einer Gaseintrittstemperatur von 350°C getrocknet. Die Luftmenge wurde so eingestellt, dass eine Austrittstemperatur von 110 +/- 10°C erhalten wurde.

Dieses Pulver wurde in einem Umluftofen bei einer Temperatur von 445°C für 1 Stunde behandelt, bis sich ein Mischoxid bildete. Das Mischoxid wurde als wässrige Suspension durch eine Zweistoffdüse auf einen kugelförmigen Styroporträger gesprüht und bei 60°C im Luftstrom getrocknet. Zur Homogenisierung der Pellets wurden diese umgewälzt. Zur Verfestigung der aufgebrachten Aktivmasse wurde das erhaltene Gut in Gegenwart von Sauerstoff bei 520°C für 5 Stunden erwärmt.

Beispiel 2

Der Katalysator des Beispiels 1 wurde mit einem Gemisch der Zusammensetzung von 7,3 vol% Propen (chemical grade), 60 vol% Luft und Inertgas beaufschlagt. Bei einer Badtemperatur von 318°C und einer Kontaktzeit von 2,0s wurde bei einem Umsatz von 92%, Acrolein und Acrylsäure mit einer Selektivität von 95% erhalten.

Vergleichsbeispiel 3

Der Katalysator wurde entsprechend des Beispiels 1 hergestellt. Als Träger wurde statt der Styroporkugel ein Aluminaträger benutzt, der sich nicht entfernen lässt. Der erhaltene Katalysator besaß dieselbe geometrische Form. Bei einer um 12 °C höheren Badtemperatur musste die Reaktionszeit, um den Faktor 1,35 verlängert werden, um vergleichbare Umsätze zu erhalten. Die Selektivität von Acrolein und Acrylsäure betrug dabei 94%.

**Patentansprüche**

**1.** Aus hohlen Formen bestehende Mischoxidkatalysatoren der allgemeinen Formel

$$(Mo_{12}Bi_aC_b(Co+Ni)_cD_dE_eF_fG_gH_h)O_x \qquad (I),$$

in der bedeuten

C: Eisen,
D: mindestens eines der Elemente ausgewählt aus der Gruppe, bestehend aus W, P,
E: mindestens eines der Elemente ausgewählt aus der Gruppe, bestehend aus Li, K, Na, Rb, Cs, Mg, Ca, Ba, Sr,
F: mindestens eines der Elemente ausgewählt aus der Gruppe, bestehend aus Ce, Mn, Cr, V
G: mindestens eines der Elemente ausgewählt aus der Gruppe, bestehend aus Nb, Se, Te, Sm, Gd, La, Y, Pd, Pt, Ru, Ag, Au
H: mindestens eines der Elemente ausgewählt aus der Gruppe, bestehend aus Si, Al, Ti, Zr
und
$a = 0,5 - 5,0$
$b = 0,5 - 5,0$
$c = 2 - 15$
$d = 0,01 - 5,0$
$e = 0,001 - 2$
$f = 0,001 - 5$
$g = 0 - 1,5$
$h = 0 - 800$,
und
$x$ = Zahl, die von der Wertigkeit und Häufigkeit der von Sauerstoff verschiedenen Elemente bestimmt wird, umfassend Hohlkugeln mit einem Durchmesser von 0,5-5 mm.

2. Mischoxidkatalysatoren gemäß Anspruch 1, umfassend eine gasundurchlässige hohle Form.

3. Mischoxidkatalysator gemäß Anspruch 1, umfassend eine gasdurchlässige hohle Form.

4. Mischoxidkatalysator gemäß den Ansprüchen 1 bis 3, umfassend eine aus mehreren Schichten bestehende hohle Form.

5. Verfahren zur Herstellung von aus hohlen Formen bestehenden Mischoxid-Katalysatoren gemäß den Ansprüchen 1 bis 4, umfassend, dass man Lösungen von Verbindungen der in den Mischoxidkatalysatoren gemäß Formel I enthaltenden Elemente vermischt, Kopräzipitate herstellt, den erhaltenen Feststoff isoliert, gegebenenfalls trocknet und kalziniert und den erhaltenen feinteiligen Feststoff wahlweise zusammen mit einem Bindemittel in Form einer Suspension als Schicht auf einen Träger, der aus organischem Material besteht und die Form einer Kugel hat, aufbringt, und dieses organische Material nach oder während der Verfestigung der auf dieses aufgebrachten Schicht entfernt.

6. Verfahren gemäß Anspruch 5, bei dem man das Katalysatorvorprodukt nach dem Aufbringen des feinteiligen Feststoffs trocknet und eine Temperaturbehandlung im Temperaturbereich von 450 bis 600°C in Gegenwart von Sauerstoff anschließt.

7. Verfahren zur Herstellung von Aldehyden und Säuren durch Oxidation von Olefinen oder methylierten Aromaten mit Luft oder Sauerstoff in Gegenwart von Inertgasen, Wasserdampf oder Abgasen aus der Reaktion bei erhöhten Temperaturen, **dadurch gekennzeichnet, dass** man einen Katalysator gemäß Anspruch 1 einsetzt.

8. Verfahren gemäß Anspruch 7, bei dem man Katalysatoren gemäß den Ansprüchen 1 bis 4 einsetzt.

9. Verfahren gemäß Anspruch 7 oder 8, **dadurch gekennzeichnet, dass** man Acrolein und Acrylsäure aus Propen gewinnt.

10. Verfahren gemäß Anspruch 7 oder 8, **dadurch gekennzeichnet, dass** man Methacrolein und Methacrylsäure aus Isobuten gewinnt.

11. Verfahren gemäß Anspruch 7 oder 8, **dadurch gekennzeichnet, dass** man Benzaldehyd und Benzoesäure aus Toluol gewinnt.

12. Verfahren gemäß den Ansprüchen 7 bis 11, **dadurch gekennzeichnet, dass** man die den Katalysator mit einem Reaktionsgasgemisch beaufschlagt, das Olefin oder methylierte Aromaten, Luft, Inertgase in einem Verhältnis von 1:6-9:3-18 enthält.

**Claims**

1. Mixed oxide catalysts which consist of hollow shapes and are of the general formula

$$(Mo_{12}Bi_aC_b(Co+Ni)_cD_dE_eF_fG_gH_h)O_x \qquad (I)$$

in which

C: iron,
D: at least one of the elements selected from the group consisting of W, P,
E: at least one of the elements selected from the group consisting of Li, K, Na, Rb, Cs, Mg, Ca, Ba, Sr,
F: at least one of the elements selected from the group consisting of Ce, Mn, Cr, V,
G: at least one of the elements selected from the group consisting of Nb, Se, Te, Sm, Gd, La, Y, Pd, Pt, Ru, Ag, Au,
H: at least one of the elements selected from the group consisting of Si, Al, Ti, Zr,
and
$a = 0.5 - 5.0$
$b = 0.5 - 5.0$
$c = 2 - 15$
$d = 0.01 - 5.0$
$e = 0.001 - 2$
$f = 0.001 - 5$
$g = 0 - 1.5$
$h = 0 - 800$,
and
$x$ = number which is determined by the valency and frequency of the elements other than oxygen, comprising hollow spheres having a diameter of 0.5-5 mm.

2. Mixed oxide catalysts according to Claim 1, comprising a gas-impermeable hollow shape.

3. Mixed oxide catalysts according to Claim 1, comprising a gas-permeable hollow shape.

4. Mixed oxide catalysts according to Claims 1 to 3, comprising a hollow shape consisting of a plurality of layers.

5. Process for preparing mixed oxide catalysts consisting of hollow shapes according to Claims 1 to 4, comprising mixing solutions of compounds of the elements present in the mixed oxide catalysts of the formula I, preparing coprecipitates, isolating the resulting solid, optionally drying and calcining it, and applying the resulting finely divided solid, optionally together with a binder, in the form of a suspension as a layer to a support which consists of organic material and has the shape of a sphere, and removing this organic material during or after the solidification of the layer applied to it.

6. Process according to Claim 5, in which the catalyst precursor, after the application of the finely divided solid, is dried, which is followed by a thermal treatment in the temperature range of 450 to 600°C in the presence of oxygen.

7. Process for preparing aldehydes and acids by oxidizing olefins or methylated aromatics with air or oxygen in the presence of inert gases, steam or offgases from the reaction at elevated temperatures, **characterized in that** a catalyst according to Claim 1 is used.

8. Process according to Claim 7, in which catalysts according to Claims 1 to 4 are used.

9. Process according to Claim 7 or 8, **characterized in that** acrolein and acrylic acid are obtained from propene.

10. Process according to Claim 7 or 8, **characterized in that** methacrolein and methacrylic acid are obtained from isobutene.

**11.** Process according to Claim 7 or 8, **characterized in that** benzaldehyde and benzoic acid are obtained from toluene.

**12.** Process according to Claims 7 to 11, **characterized in that** the catalyst is contacted with a reaction gas mixture which comprises olefin or methylated aromatics, air, inert gases in a ratio of 1:6-9:3-18.

**Revendications**

**1.** Catalyseurs d'oxyde mixte constitués par des formes creuses, présentant la formule générale

$$(Mo_{12}Bi_aC_b(Co+Ni)_cD_dE_eF_fG_gH_h)O_x \qquad (I)$$

, dans laquelle

C : signifie fer,
D : signifie au moins un des éléments choisis dans le groupe constitué par W et P,
E : signifie au moins un des éléments choisis dans le groupe constitué par Li, K, Na, Rb, Cs, Mg, Ca, Ba, Sr,
F : signifie au moins un des éléments choisis dans le groupe constitué par Ce, Mn, Cr, V
G : signifie au moins un des éléments choisis dans le groupe constitué par Nb, Se, Te, Sm, Gd, La, Y, Pd, Pt, Ru, Ag, Au
H : signifie au moins un des éléments choisis dans le groupe constitué par Si, Al, Ti, Zr et
a = 0,5-5,0
b = 0,5-5,0
c = 2-15
d = 0,01-5,0
e = 0,001-2
f = 0,001-5
g = 0-1,5
h = 0-800, et
x = un nombre qui est déterminé par la valence et la fréquence des éléments différents de l'oxygène, comprenant des billes creuses présentant un diamètre de 0,5-5 mm.

**2.** Catalyseurs d'oxyde mixte selon la revendication 1, comprenant une forme creuse imperméable au gaz.

**3.** Catalyseur d'oxyde mixte selon la revendication 1, comprenant une forme creuse perméable au gaz.

**4.** Catalyseur d'oxyde mixte selon les revendications 1 à 3, comprenant une forme creuse constituée par plusieurs couches.

**5.** Procédé pour la préparation de catalyseurs d'oxyde mixte constitués par des formes creuses selon les revendications 1 à 4, comprenant le mélange de solutions de composés des éléments contenus dans les catalyseurs d'oxyde mixte selon la formule I, la préparation de coprécipités, l'isolement, le cas échéant le séchage et la calcination du solide obtenu et l'application du solide finement divisé obtenu, éventuellement conjointement avec un liant sous forme d'une suspension, en tant que couche sur un support qui est constitué par un matériau organique et qui présente la forme d'une bille et l'élimination de ce matériau organique après ou pendant la solidification de la couche appliquée sur celui-ci.

**6.** Procédé selon la revendication 5, dans lequel on sèche le précurseur catalytique après l'application du solide finement divisé et un traitement thermique dans la plage de température de 450 à 600°C en présence d'oxygène est effectué ensuite.

**7.** Procédé pour la préparation d'aldéhydes et d'acides par oxydation d'oléfines ou d'aromatiques méthylés avec de l'air ou de l'oxygène en présence de gaz inertes, de vapeur d'eau ou d'effluents gazeux provenant de la réaction à des températures augmentées, **caractérisé en ce qu'**on utilise un catalyseur selon la revendication 1.

**8.** Procédé selon la revendication 7, dans lequel on utilise des catalyseurs selon les revendications 1 à 4.

**9.** Procédé selon la revendication 7 ou 8, **caractérisé en ce qu'**on produit de l'acroléine et de l'acide acrylique à partir de propène.

10. Procédé selon la revendication 7 ou 8, **caractérisé en ce qu'**on produit de la méthacroléine et de l'acide méthacrylique à partir d'isobutène.

11. Procédé selon la revendication 7 ou 8, **caractérisé en ce qu'**on produit du benzaldéhyde et de l'acide benzoïque à partir de toluène.

12. Procédé selon les revendications 7 à 11, **caractérisé en ce qu'**on soumet le catalyseur à un mélange gazeux réactionnel qui contient une oléfine ou des aromatiques méthylés, de l'air, des gaz inertes dans un rapport de 1:6-9:3-18.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 821999 A
- WO 2005063673 A
- WO 2005035115 A
- DE 10344149
- DE 19933450
- US 4537874 A
- DE 10344149 A1
- JP 5277381 A
- GB 2055787 A
- GB 1442566 A

**In der Beschreibung aufgeführte Nicht-Patentliteratur**

- neue Hochporöse Metallische Werkstoffe. *Ingenieur-Werkstoffe,* 1998, vol. 4, 36-38